Europäisches Patentamt

European Patent Office

Office européen des brevets

(11) Publication number: **0 036 272**
**B1**

# EUROPEAN PATENT SPECIFICATION

(45) Date of publication of patent specification: **09.01.85**

(51) Int. Cl.⁴: **A 61 K 6/08**

(21) Application number: **81300921.4**

(22) Date of filing: **05.03.81**

(54) Permanent dental restorative compositions and methods of using them.

(30) Priority: **07.03.80 US 128010**

(43) Date of publication of application:
**23.09.81 Bulletin 81/38**

(45) Publication of the grant of the patent:
**09.01.85 Bulletin 85/02**

(84) Designated Contracting States:
**AT BE CH DE FR GB IT LI NL SE**

(56) References cited:
**DE-A-1 544 925**
**DE-A-1 933 917**
**GB-A- 982 814**

**Ullmanns Encyklopädie, Vol. 10, pp. 8-9 (1975),
4th Ed.**

(73) Proprietor: **Rohm and Haas Company
Independence Mall West
Philadelphia, Pennsylvania 19105 (US)**

(72) Inventor: **Fellman, Robert Paul
115 Hilltop Court
Langhorne Pennsylvania 19047 (US)**
Inventor: **Myers, Robert Marshall
3139 Sheffield Place
Holland Pennsylvania 18966 (US)**
Inventor: **Hurwitz, Marvin Joseph
8267 Thompson Road
Elkins Park Pennsylvania 19117 (US)**
Inventor: **Slack, Gerald Frederick
Ridge Road - R.D. No. 6
Vincentown New Jersey 08088 (US)**

(74) Representative: **Angell, David Whilton et al
Rohm and Haas Company Patent Department
Chesterfield House Bloomsbury Way
London WC1A 2TP (GB)**

Courier Press, Leamington Spa, England.

**0 036 272**

**Description**

The field of this invention is the composite dental restorative material art, and permanent dental restorations made from such materials.

Compatibility of Various Materials with Oral Tissues
1: *The Components in Composite Restorations*

Bowen, J. Dent Res. 58 (5): 1493—1503, May 1979, summarizes the current state of the art with respect to composite restorative materials used for dentistry. This art is different from the art of dental prostheses, appliances, and dentures, since the restorative material must be castable in place.

DE—A—1 933 917 discloses adhesive compositions useful for dental prostheses, which comprise a liquid phase containing methyl methacrylate and crosslinking agent, a mixture of inorganic substances and organic polymer together with polymerization catalyst.

In GB—A—982 814 dental compositions are also disclosed which comprise methyl methacrylate and thickener, insoluble organic polymer and a catalyst.

Ullmanns Encyclopadie der Technischen chemie, 4th Edition, Vol. 10, Verlag Chemie Weinheim, 1975 pages 8 and 9 describes a technique of obtaining dental prostheses by mixing (i) insoluble polymethacrylate (ii) methacrylate monomer (iii) crosslinking agent and (iv) catalyst.

We have now unexpectedly found that certain compositions containing liquid monomer, particulate substance and initiator of the types and proportions as defined in claim 1 can form permanent dental restorations having particularly desirable compressive strength and/or rotary wear values. The invention also provides methods for making such permanent dental restorations and also the restorations themselves. The compositions of the invention may be used for preparing permanent dental restorations which can be cast in a dental office in a short period of time, or in a dental laboratory.

According to the invention there is provided a composition, suitable for preparing permanent dental restorations, comprising (a) a liquid monomer system, (b) particulate substance and (c) a redox initiator system characterised in that the composition comprises from 25 to 80 parts by weight of (a) liquid monomer system comprising methyl methacrylate and from 15 to 50% by weight, based on liquid monomer system, of at least one polyethylenically unsaturated crosslinking monomer; and from 20 to 75 parts by weight of (b) a mixture of (1) organic polymeric particulate substance insoluble in said liquid monomer system comprising at least one of poly(ethylene-co-chlorotrifluoroethylene), poly-(vinylidene fluoride), nylon, and polyacetal, and (2) inorganic particulate substance insoluble in said liquid monomer system the weight ratio of organic polymeric particulate substance to inorganic particulate substance being from 1:5 to 1:1; and that said composition, when cured, has a compressive strength of at least 0.2068 G Pa (30,000 psi) and/or a rotary wear of at least 20 hours/25.4 $\mu$m (20 hours/mil).

In another aspect, the invention comprises permanent dental restorations having high compressive strength and wear resistance cast from such compositions, and methods of making such permanent dental restorations comprising mixing the liquid monomer system, the solid particulate system, and the two components of the redox initiator system, filling a mould with the mixture, allowing the composition to set, and then to cure.

The compositions of the invention are such that they provide, and the permanent restorations, particularly crowns, have, a minimum compressive strength of 0.2068 G Pa (30,000 psi) and/or a rotary wear value of at least 20 hours/25.4 $\mu$m (20 hours/mil). In this specification and claims, these values are obtained by the methods described in example 1, and "high compressive strength and wear resistance" preferably means having the above values.

In this specification and claims the term "permanent" means long lasting, as opposed to temporary dental restorations. Temporary restorations are only designed to last approximately six months or less, whereas the restorations of the invention are designed to last longer, generally for the life of the patient, although these "permanent" restorations can be removed by a dentist if necessary. The term "restorations" refers to crowns, bridges, fillings, repairs to damage caused by trauma, repairs to existing crowns, and cosmetic repair in general. "Restorative material" refers to material used for such restorations. The term "crown" refers to the whole tooth surface rather than selected portions thereof.

As previously mentioned, the solid particulate system or filler is comprised of the mixture of both organic polymeric and inorganic particulate substances, each of said substances being insoluble in the liquid monomer system. The organic polymeric particulate substance is at least one of poly(ethylene-co-chlorotrifluoroethylene), poly(vinylidene fluoride), polyacetal and nylon. Examples of suitable nylon are nylon 6, nylon 610, nylon 66, and nylon 11.

The inorganic particulate substance is preferably hydroxyapatite or stearate-coated calcium carbonate, but calcium carbonate generally, glass beads, quartz, silica, talc, clay, calcium sulfate, glass fibers, and combinations of any of the aforementioned inorganic substances can be used. 20 to 75 parts by weight of the solid particulate system is used with 25 to 80 parts, preferably 50 to 70 parts, of the liquid monomer system. The preferred amount of solid particulate system is about 30 to 50 parts by

2

weight. The particle size of the inorganic particulate substance is preferably about 0.075 to about 90 $\mu$m and the particle size of the organic polymeric particulate substance is preferably about 25 to 75 $\mu$m. The weight ratio of organic polymeric particulate substance to inorganic particulate substance is 1:5 to 1:1, preferably 1:3 to 2:3.

Although a wide range of redox initiator systems are known in the art, and are suitable, two particularly suitable systems are a combination of benzoyl peroxide and N,N-bis-(hydroxyethyl)-p-toluidine, and a second system comprised of t-butylperoxy maleic acid and calcium hydroxide. Other suitable combinations are described by J. M. Antonucci et al., in J. Dent. Res., 58 (9), 1887—99. Preferred amounts of redox pair to be used are 0.5 to 10 parts by weight, more preferably 1 to 5 parts by weight, per 100 parts of the resin system. The redox pair consists of an oxidizing agent and reducing agent. In practice, it is preferred to include the oxidizing agent with the filler system as one component, and the reducing agent with the liquid monomer system as a second component, it being understood that the oxidizing and reducing agents are components of the redox initiator system. Further optional ingredients such as one or more of tinting agent, stabilizer to control polymerization rate, exotherm, and yellowing, fluorescing agent and x-ray opacifying agent can be included in either component.

The liquid monomer system is comprised of methyl methacrylate and 15 to 50% by weight, preferably 20 to 50% by weight, based on liquid monomer system, of at least one polyethylenically unsaturated crosslinking monomer. The amount and type of polyethylenically unsaturated monomer may be selected so as to achieve curing in the desired length of time, to control exotherm temperature, and to synergistically contribute to the wear resistance and compressive strength of the resultant permanent dental crown. The preferred polyethylenically unsaturated monomers are polyfunctional acrylates or methacrylates or mixtures thereof, and are preferably selected from the group consisting of trimethylolpropane trimethacrylate, 2,2-bis [4-(2-hydroxy-3-methacryloxypropoxy)phenyl] propane, (bis-GMA), divinylbenzene, diallyl maleate, ethylene and butylene glycol dimethacrylate, 1,6-hexanediol dimethacrylate, butylene diacrylate, pentaerythritol tetraacrylate, and ethoxylated bis-phenol-A dimethacrylate.

Besides methyl methacylate, the composition can further include up to 10% by weight, based on liquid monomer system, of at least one different monoethylenically unsaturated monomer such as, for example, octafluoropentyl methacrylate, hydroxyethyl methacrylate, hexafluoroisopropyl methacrylate, pentafluoropropyl acrylate, pentafluoropropyl methacrylate, heptafluorobutyl acrylate, heptafluorobutyl methacrylate, and butyl methacrylate.

The composition can also include a thickener polymer dissolved, for example, in the liquid monomer system. Such thickener polymer can be any polymer soluble in the liquid monomer system, for example $C_1$ to $C_4$ alkyl esters of acrylic acid and methacrylic acid. Alternatively, thickeners insoluble in the liquid monomer system such as very finely divided hydrophobic silicon dioxide can be included.

The permanent dental restorations of the invention may have a combination of high compressive strength and wear resistance never before achieved in the prior art, and may be competitive with amalgam, gold, or crown grade porcelain. The restorations to which this invention is most applicable are crowns and bridges, and especially crowns.

The method of making the permanent dental crowns of the invention is generally to mix the liquid monomer system, the solid particulate system, and the redox initiator system, fill a tooth-shaped mould with the mixture, and apply the filled mould to the tooth stump to which the crown is to be applied. It is important to keep the reducing agent and oxidizing agent separated until the composition is mixed at the time of use. The composition can be in the form of mixing two components, for example two pastes, a paste and a powder, or a paste and a liquid. The composition is allowed to set, and then to cure. A preferred method comprises (I) mixing (A) a first component comprising 25 to 80 parts by weight of a liquid monomer system comprised of methyl methacrylate, 15 to 15% by weight, based on liquid monomer system, of a polyethylenically unsaturated crosslinking monomer, and a small amount of a reducing agent with (B) a second component comprising 20 to 75 parts by weight of a solid particulate system comprised of a mixture of both organic polymeric and inorganic particulate substances said organic polymeric substance comprising at least one of poly(ethylene-co-chlorotrifluoroethylene), poly-(vinylidene fluoride), nylon, and polyacetal and the weight ratio of organic polymeric particulate substance to inorganic particulate substance being from 1:5 to 1:1, both of said substances being insoluble in the first component, and an oxidizing agent, (II) allowing the resultant mixture to achieve a suitable viscosity, (III) filling a toothshaped preformed mold or impression tray with thickened mixture (IV) applying the filled mold or tray to the tooth stump to which the crown is to be applied, (V) allowing the composition to set, (VI) removing the set crown from mouth and then from the mold or impression tray, the crown, when cured having a compressive strength of at least 0.2068 GPa (30,000 psi) and/or a rotary wear of at least 20 h/25.4 $\mu$m (20 hours/mil). The cured crown may be finished and polished, if necessary, and the crown may then be filled with dental cement and placed on the stump.

The temperature rise during the setting is desirably kept below 15°C so as to avoid discomfort to the patient, and is preferably within the range of 5°C to 10°C over a set time of 5 to 15 minutes.

It is preferred that the crown be prepared in a dental office by applying the composition to the tooth stump and allowing it to set, then curing the crown outside the mouth by applying heat at 80°C to 100°C for 10 to 20 minutes, then applying dental cement to the inside of the crown, then reapplying the crown to the stump. An alternative method is to leave the crown on the stump until it sets, and then allowing it to cure in place without removing it. Another method is to send a mold of the tooth stump to a dental laboratory and making the crown from the composition of the invention at the laboratory, then having the dentist at a later time apply adhesive to the stump or the crown.

The following specific examples are presented to illustrate a few embodiments of the invention, but it is to be understood that the invention is not limited thereto.

### Example 1

Test specimens were prepared by mixing in a Waring Blendor the liquid monomer system, organic and inorganic particulate substances and *redox initiator system* for 20 seconds, pouring into a disk-shaped mould and a tubular mould, and setting for 15 minutes at 37°C. The initiator system consisted of 0.42% by weight 2-N,N-bis-(hydroxyethyl)-p-toluidine and 1.74% by weight benzoyl peroxide based on the weight of the monomers. Four percent by weight of high molecular weight polymethyl methacrylate thickener was dissolved in the liquid monomer system so as to aid in achieving homogeneous mixing. Tints, radio-opacifying agents, and fluorescing agents can also be included in this particular experiment. To 35 parts insoluble particulate substances comprised of a 2:1 mixture of stearate-coated calcium carbonate and poly(ethylene-co-chlorotrifluoroethylene) (1/1) was mixed 65 parts by weight of a liquid monomer system comprised of 60% methyl methacrylate and 40% trimethylolpropane trimethacrylate. The resultant compositions after thickening, setting reaction and cure were tested for wear life and compressive strength. The wear life test consisted of rotating a cast disc sample under an off-center ceramic scriber having a cylindrical tip 1524 $\mu$m (60 mils) in diameter under a load of one kilogram/mm$^2$ at 27 rpm for 64,800 revolutions. The test is conducted at 37°C with continuous water washing. See Powell, J. M., and Dickson, G., *J. Dent. Res. 54* (Special Issue A) 134 (1975). The compressive strength test is in accordance with ASTM D-595 and consists of crushing a rod 0.635 cm (one-quarter inch) in diameter by 1.27 cm (one-half inch) along its length at 0.127 cm/min (0.05 in./minute) after presoaking for 24 hours at 37°C in water, and measuring the compressive strength immediately after removal from the water.

### Example 2

Example 1 was repeated except using as the polyethylenically unsaturated monomer ethylene glycol dimethacrylate.

### Example 3

Example 1 was repeated except using methyl methacrylate/trimethylolpropane trimethacrylate ratio of 80:20.

### Example 4

Example 1 was repeated except using methyl methacrylate/trimethylolpropane trimethacrylate ratio of 90:10.

### Example 5

Example 1 was repeated except using a ratio of 30 parts of particulate filler to 70 parts of liquid monomer and using a methyl methacrylate/trimethylolpropane trimethacrylate ratio of 80:20.

### Example 6

Example 5 was repeated except using polyvinylidene fluoride as the organic particulate filler. (Average particle size 50 $\mu$m).

### Example 7

Example 1 was repeated except using polyvinylidene fluoride as the organic particulate filler and using calcium hydroxyapatite, reagent grade, average particle size 50 $\mu$m, at a ratio of organic to inorganic particulate of 1:1, and using a ratio of 30 parts particulate filler system to 70 parts of liquid monomer system.

### Example 8

Example 1 was repeated except using nylon 66 as the organic particulate filler, using stearate-coated calcium carbonate as the inorganic particulate filler, an organic/inorganic filler ratio of 1:1, omitting thickener, and using a ratio of 30 parts particulate filler to 70 parts of liquid monomer.

### Example 9

Example 1 was repeated except using calcium carbonate having an average particle size of 15 $\mu$m, omitting thickener, and a particulate/liquid monomer ratio of 30:70.

## Example 10
Example 9 was repeated except using stearate-coated calcium carbonate which had a particle size of 0.075 $\mu$m average.

## Example 11
Example 1 was repeated except using a liquid monomer system to solid filler system ratio of 70:30, using polyvinylidene fluoride as the organic particulate, and omitting thickener.

## Example 12
Example 11 was repeated except using a filler to liquid monomer ratio of 40:60.

## Example 13
Example 12 was repeated except using a ratio of 50:50.

## Example 14
Example 7 was repeated except using calcium sulfate instead of calcium hydroxyapatite and omitting thickener.

## Example 15
Example 1 was repeated except using as the inorganic particulate filler system stearate-coated calcium carbonate and uncoated calcium carbonate in a ratio of 1:1, and omitting the thickener.

## Example 16
The results of wear life tests and compressive strength tests for Examples 1 to 15 are reported in the following table.

### TABLE

| Example No. | Wear Test Results hours/25.4 $\mu$m (hours/mil) | Compressive Strength (Thousands of Pounds Per Square Inch) | GPa |
|---|---|---|---|
| 1 | 41 | — | |
| 2 | 39 | — | |
| 3 | 44 | — | |
| 4 | 16 | — | |
| 5 | 63 | 40 | 0.275 |
| 6 | 44 | 36 | 0.248 |
| 7 | 72 | 26 | 0.179 |
| 8 | 47 | 31 | 0.214 |
| 9 | 46 | 30 | 0.207 |
| 10 | 32 | 30 | 0.207 |
| 11 | 72 | 36 | 0.248 |
| 12 | 56 | 30 | 0.207 |
| 13 | 43 | 26 | 0.179 |
| 14 | 44 | 29 | 0.200 |
| 15 | 61 | 35 | 0.241 |

# 0 036 272

## Claims

1. A composition, suitable for preparing permanent dental restorations, comprising (a) a liquid monomer system, (b) particulate substance and (c) a redox initiator system characterised in that the composition comprises from 25 to 80 parts by weight of (a) liquid monomer system comprising methyl methacrylate and from 15 to 50% by weight, based on liquid monomer system, of at least one polyethylenically unsaturated crosslinking monomer; and from 20 to 75 parts by weight of (b) a mixture of (1) organic polymeric particulate substance insoluble in said liquid monomer system comprising at least one of poly(ethylene-co-chlorotrifluoroethylene), poly(vinylidene fluoride), nylon, and polyacetal, and (2) inorganic particulate substance insoluble in said liquid monomer system the weight ratio of organic polymeric particulate substance to inorganic particulate substance being from 1:5 to 1:1; and that said composition, when cured, has a compressive strength of at least 0.2068 GPa (30,000 psi) and/or a rotary wear of at least 20 hours/25.4 $\mu$m (20 hours/mil).

2. A composition as claimed in claim 1 characterised in that the composition is in the form of two packages, one comprising component (a) and the reducing agent of component (c), the other comprising component (b) and the oxidising agent of component (c).

3. A composition as claimed in claim 1 or 2 characterised in that it contains 30 to 50 parts by weight of said mixture of (1) and (2).

4. A composition as claimed in any of claims 1 to 3 characterised in that the particle size of said inorganic particulate substance is from 0.075 to 90 $\mu$m.

5. A composition as claimed in any of claims 1 to 4 characterised in that the particle size of said organic polymeric particulate substance is from 25 to 75 $\mu$m.

6. A composition as claimed in any of claims 1 to 5 characterised in that said inorganic particulate substance comprises at least one of hydroxyapatite, calcium carbonate, stearate-coated calcium carbonate, glass beads, quartz, silica, talc, clay, calcium sulfate and glass fiber.

7. A composition as claimed in any of claims 1 to 6 characterised in that the polyethylenically unsaturated monomer comprises from 20 to 50% by weight of the liquid monomer system.

8. A composition as claimed in any of claims 1 to 7 characterised in that said polyethylenically unsaturated monomer comprises at least one polyfunctional acrylate and/or methacrylate.

9. A composition as claimed in any of claims 1 to 7 characterised in that the polyethylenically unsaturated monomer comprises at least one of trimethylolpropane trimethacrylate, ethylene glycol dimethacrylate, 2,2-bis (4-[2-hydroxy-3-methacryloxypropoxy] phenyl) propane, divinylbenzene, diallyl maleate, butylene glycol dimethacrylate, 1,6-hexanediol dimethacrylate, butylene diacrylate, pentaerythritol tetraacrylate, and ethoxylated bis-phenol-A dimethacrylate.

10. A composition as claimed in any of claims 1 to 9 characterised in that it further includes up to 10 percent by weight, based on liquid monomer system, of at least one monoethylenically unsaturated monomer other than methyl methacrylate.

11. A composition as claimed in claim 10 characterised in that said monoethylenically unsaturated monomer comprises at least one of octafluoropentyl methacrylate, hydroxyethyl methacrylate, hexafluoroisopropyl methacrylate, pentafluoropropyl acrylate, pentafluoropropyl methacrylate, heptafluorobutyl acrylate, heptafluorobutyl methacrylate and butyl methacrylate.

12. A composition as claimed in any of claims 1 to 11 characterised in that (c) comprises a combination of benzoyl peroxide and an aromatic amine or t-butylperoxy maleic acid and calcium hydroxide.

13. A composition as claimed in any of claims 1 to 12 characterised in that it further includes one or more of thickener tinting agent, stabilizer to control polymerization rate and exotherm, stabilizer to prevent yellowing, fluorescing agent, and x-ray opacifying agent.

14. A permanent dental restoration prepared from a composition according to any of claims 1 to 13.

15. A method of making a permanent dental restoration which comprises filling a mould with a composition according to any of claims 1 to 13 allowing the composition to set or setting it and then curing the composition or allowing it to cure.

16. A method of making permanent dental crowns which comprise (I) mixing (A) a first component comprising from 25 to 80 parts by weight of a liquid monomer system comprising methyl methacrylate, from 15 to 50% by weight, based on liquid monomer system, of at least one polyethylenically unsaturated crosslinking monomer; and reducing agent; with (B) a second component comprising from 20 to 75 parts by weight of a mixture of (1) organic polymeric particulate substance comprising at least one of poly(ethylene-co-chlorotrifluoroethylene), poly(vinylidene fluoride), nylon, and polyacetal, and (2) inorganic particulate substances, the weight ratio of organic polymeric particulate substance to inorganic particulate substance being from 1:5 to 1:1, both of said substances being insoluble in said first component, and oxidizing agent; (II) allowing the resultant mixture to achieve a suitable viscosity, (III) filling a tooth-shaped preformed mould or impression tray with the thickened mixture, and (IV) applying the filled mould or tray to the tooth-stump to which the crown is to be applied, (V) allowing the composition to set and (VI) to removing the resultant set crown from the mouth and then from the mould or impression tray, the crown, when cured, having a compression strength of at least 0.2068 GPa (30,000 psi) and/or rotary wear of at last 20 h/25.4 $\mu$m (20 hours/mil).

6

17. A method as claimed in Claim 16 as applied to a composition according to any of claims 3 to 13.

**Patentansprüche**

1. Zur Herstellung permanenter Zahnreparaturen geeignete Masse aus (a) einem flüssigen Monomersystem, (b) einer in Form von Einzelteilchen vorliegenden Substanz und (c) einem Redoxinitiatorsystem, dadurch gekennzeichnet, daß die Masse 25 bis 80 Gew.-Teile (a) eines flüssigen Monomersystems aus Methylmethacrylat und 15 bis 50 Gew.-%, bezogen auf das flüssige Monomersystem, wenigstens eines polyethylenisch ungesättigten vernetzenden Monomeren und 20 bis 75 Gew.-Teile (b) einer Mischung aus (1) einer organischen polymeren in Form von Einzelteilchen vorliegenden Substanz, die in dem flüssigen Monomersystem unlöslich ist und aus wenigstens einer der Substanzen Poly(ethylen-co-chlortrifluorethylen), Poly(vinylidenfluorid), Nylon und Polyacetal besteht, und (2) einer anorganischen in Form von Einzelteilchen vorliegenden Substanz, die in dem flüssigen Monomersystem unlöslich ist, aufweist, wobei das Gewichtsverhältnis der organischen Polymeren in Form von Einzelteilchen vorliegenden Substanz zu der anorganischen in Form von Einzelteilchen vorliegenden Substanz 1:5 bis 1:1 beträgt, und wobei die Masse nach dem Härten eine Druckfestigkeit von wenigstens 0,2068 G Pa (30.000 psi) und/oder einen Rotationsabrieb von wenigstens 20 h/25,4 $\mu$m (20 h/mil) aufweist.

2. Masse nach Anspruch 1, dadurch gekennzeichnet, daß sie in Form von zwei Packungen vorliegt, wobei eine die Komponente (a) und das Reduktionsmittel der Komponente (c) und die andere die Komponente (b) und das Oxidationsmittel der Komponente (c) aufweist.

3. Masse nach Anspruch 1 oder 2, dadurch gekennzeichnet, daß sie 30 bis 50 Gew.-Teile der Mischung von (1) und (2) aufweist.

4. Masse nach einem der Ansprüche 1 bis 3, dadurch gekennzeichnet, daß die Teilchengröße der anorganischen in Form von Einzelteilchen vorliegenden Substanz 0,075 bis 90 $\mu$m beträgt.

5. Masse nach einem der Ansprüche 1 bis 4, dadurch gekennzeichnet, daß die Teilchengröße der organischen polymeren in Form von Einzelteilchen vorliegenden Substanz 25 bis 75 $\mu$m beträgt.

6. Masse nach einem der Ansprüche 1 bis 5, dadurch gekennzeichnet, daß die anorganische in Form von Einzelteilchen vorliegende Substanz aus wenigstens einer der Substanzen Hydroxyapitit, Calciumcarbonat, mit Stearat überzogenem Calciumcarbonat, Glaskügelchen, Quartz, Siliziumdioxid, Talk, Ton, Calciumsulfat und Glasfasern besteht.

7. Masse nach einem der Ansprüche 1 bis 6, dadurch gekennzeichnet, daß das polyethylenisch ungesättigte Monomere 20 bis 50 Gew.-% des flüssigen Monomersystems ausmacht.

8. Masse nach einem der Ansprüche 1 bis 7, dadurch gekennzeichnet, daß das polyethylenisch ungesättigte Monomere wenigstens ein polyfunktionelles Acrylat und/oder Methacrylat aufweist.

9. Masse nach einem der Ansprüche 1 bis 7, dadurch gekennzeichnet, daß das polyethylenisch ungesättigte Monomere aus wenigstens einer der Substanzen Trimethylolpropantrimethacrylat, Ethylenglykoldimethacrylat, 2,2-Bis-(4-[2-hydroxy-3-methacryloxypropoxy]phenyl)propan, Divinylbenzol, Diallylmaleat, Butylenglykoldimethacrylat, 1,6-Hexandioldimethacrylat, Butylendiacrylat, Pentaerythrittetraacrylat sowie ethoxyliertem Bis-phenol-A-dimethacrylat besteht.

10. Masse nach einem der Ansprüche 1 bis 9, dadurch gekennzeichnet, daß sie außerdem bis zu 10 Gew.-%, bezogen auf das flüssige Monomersystem, wenigstens ein anderes monoethylenisch ungesättigtes Monomeres als Methylmethacrylat enthält.

11. Masse nach Anspruch 10, dadurch gekennzeichnet, daß das monoethylenisch ungesättigte Monomere aus wenigstens einer der Substanzen Octafluorpentylmethacrylat, Hydroxyethylmethacrylat, Hexafluorisopropylmethacrylat, Pentafluorpropylacrylat, Pentafluorpropylmethacrylat, Heptafluorbutylacrylat, Heptafluorbutylmethacrylat sowie Butylmethacrylat besteht.

12. Masse nach einem der Ansprüche 1 bis 11, dadurch gekennzeichnet, daß (c) aus einer Kombination aus Benzoylperoxid und einem aromatischen Amin oder t-Butylperoxymaleinsäure und Calciumhydroxid besteht.

13. Masse nach einem der Ansprüche 1 bis 12, dadurch gekennzeichnet, daß sie außerdem eine oder mehrere der folgenden Substanzen enthält: ein Eindickungsmittel, ein Färbemittel, ein Stabilisierungsmittel zur Steuerung der Polymerisationsgeschwindigkeit sowie der exothermen Reaktion, ein Stabilisierungsmittel zur Verhinderung eines Gelbwerdens, ein fluoreszierendes Mittel sowie ein Röntgenstrahlentrübungsmittel.

14. Permanente Zahnreparatur, hergestellt aus einer Masse gemäß einem der Ansprüche 1 bis 13.

15. Verfahren zur Herstellung einer permanenten Zahnreparatur, dadurch gekennzeichnet, daß eine Form mit einer Masse gemäß einem der Ansprüche 1 bis 13 gefüllt wird, die Masse sich verfestigen gelassen wird oder verfestigt wird und dann die Masse härten gelassen oder gehärtet wird.

16. Verfahren zur Herstellung von permanenten Zahnkronen, dadurch gekennzeichnet, daß (I) (A) eine erste Komponente aus 25 bis 80 Gew.-Teilen eines flüssigen Monomersystems aus Methylmethacrylat, 15 bis 50 Gew.-%, bezogen auf das flüssige Monomersystem, wenigstens eines polyethylenisch ungesättigten vernetzenden Monomeren und ein Reduktionsmittel mit (B) einer zweiten Komponente

**0 036 272**

aus 20 bis 75 Gew.-Teilen einer Mischung aus (1) einer organischen polymeren Substanz in Form von Einzelteilchen aus wenigstens einer der Substanzen Poly(ethylen-co-chlorotrifluorethylen), Poly(vinylidenfluorid), Nylon und Polyacetal, und (2) einer anorganischen Substanz in Form von Einzelteilchen vermischt wird, wobei das Gew.-Verhältnis der organischen polymeren Substanz in Form von Einzelteilchen zu der anorganischen Substanz in Form von Einzelteilchen 1:5 bis 1:1 beträgt, und wobei beide dieser Substanzen in der ersten Komponente und im Oxidationsmittel unlöslich sind, (II) die erhaltene Mischung auf eine geeignete Viskosität kommen gelassen wird, (III) eine zahnförmige vorgeformte Form oder Vertiefung mit der eingedickten Mischung gefüllt wird und (IV) die gefüllte Form oder die Vertiefung auf den Zahnstumpf, auf welche die Krone aufgebracht werden soll, aufgebracht wird, (V) die Masse sich verfestigen gelassen wird und (VI) die erhaltene verfestigte Krone aus dem Mund und dann aus der Form oder der Vertiefung entfernt wird, wobei die Krone in gehärtetem Zustand eine Druckfestigkeit von wenigstens 0,2068 G Pa (30.000 psi) und/oder einen Rotationsabrieb von wenigstens 20 h/25,4 $\mu$m (20 h/mil) aufweist.

17. Verfahren nach Anspruch 16, dadurch gekennzeichnet, daß es auf eine Masse gemäß einem der Ansprüche 3 bis 13 angewendet wird.

**Revendications**

1. Une composition convenant pour la préparation de restaurations dentaires permanentes comprenant (a) un système monomère liquide, (b) une substance particulaire et (c) un système amorceur redox, la composition étant caractérisée en ce qu'elle comprend 25 à 80 parties en poids de (a) un système monomère liquide comprenant du méthacrylate de méthyle et de 15 à 50 % en poids, par rapport au système monomère liquide, d'au moins un monomère réticulant à insaturation polyéthylénique, et de 20 à 75 parties en poids de (b) un mélange de (1) une substance particulaire organique polymère insoluble dans ledit système monomère liquide comprenant au moins un de: poly(éthylène-co-chlorotrifluoroéthylène), poly(fluorure de vinylidène), nylon et polyacétal, et (2) une substance particulaire minérale insoluble dans ledit système monomère liquide, le rapport pondéral de la substance particulaire organique polymère à la substance particulaire minérale étant de 1/5 à 1/1; et en ce que ladite composition, lorsqu'elle est durcie, a une résistance à la compression d'au moins 0,2068 GPa (30 000 psi) et/ou une usure par rotation d'au moins 20 heures/25,4 $\mu$m (20 heures/mil).

2. Une composition comme revendiqué dans la revendication 1, caractérisée en ce que la composition est sous forme de deux emballages, l'un comprenant le composant (a) et l'agent réducteur du composant (c), l'autre comprenant le composant (b) et l'agent oxydant du composant (c).

3. Une composition comme revendiqué dans la revendication 1 ou 2, caractérisée en ce qu'elle contient 30 à 50 parties en poids dudit mélange de (1) et (2).

4. Une composition comme revendiqué dans l'une quelconque des revendications 1 à 3, caractérisée en ce que la taille des particules de ladite substance particulaire minérale est de 0,075 à 90 $\mu$m.

5. Une composition comme revendiqué dans l'une quelconque des revendications 1 à 4, caractérisée en ce que la taille des particules de ladite substance particulaire organique polymère est de 25 à 75 $\mu$m.

6. Une composition comme revendiqué dans l'une quelconque des revendications 1 à 5, caractérisée en ce que ladite substance particulaire minérale comprend au moins un de: l'hydroxy-apatite, du carbonate de calcium, du carbonate de calcium enrobé de stéarate, des perles de verre, du quartz, de la silice, du talc, de l'argile, du sulfate de calcium et des fibres de verre.

7. Une composition comme revendiqué dans l'une quelconque des revendications 1 à 6, caractérisée en ce que le monomère à insaturation polyéthylénique constitue 20 à 50 % du poids du système monomère liquide.

8. Une composition comme revendiqué dans l'une quelconque des revendications 1 à 7, caractérisée en ce que ledit monomère à insaturation polyéthylénique comprend au moins un acrylate et/ou méthacrylate polyfonctionnel.

9. Une composition comme revendiqué dans l'une quelconque des revendications 1 à 7, caractérisée en ce que le monomère à insaturation polyéthylénique comprend au moins un de: triméthacrylate de triméthylolpropane, diméthacrylate d'éthylèneglycol, 2,2-bis[4-(2-hydroxy-3-méthacryloxypropoxy)phényl]propane, divinylbenzène, maléate de diallyle, diméthacrylate de butylèneglycol, diméthacrylate de 1,6-hexanediol, diacrylate de butylène, tétraacrylate de pentaérythritol et diméthacrylate de bis-phénol A éthoxylé.

10. Une composition comme revendiqué dans l'une quelconque des revendications 1 à 9, caractérisée en ce qu'elle comprend de plus jusqu'à, 10 % en poids, par rapport au système monomère liquide, d'au moins un monomère à insaturation monoéthylénique autre que le méthacrylate de méthyle.

11. Une composition comme revendiqué dans la revendication 10, caractérisée en ce que ledit monomère à insaturation monoéthylénique comprend au moins un de: méthacrylate d'octafluoropentyle, méthacrylate d'hydroxyéthyle, méthacrylate d'hexafluoroisopropyle, acrylate de pentafluoropropyle, méthacrylate de pentafluoropropyle, acrylate d'heptafluorobutyle, méthacrylate d'heptafluorobutyle et méthacrylate de butyle.

8

12. Une composition comme revendiqué dans l'une quelconque des revendications 1 à 11, caractérisée en ce que (c) elle comprend une combinaison de peroxyde de bensoyle et d'une amine aromatique ou d'acide tert-butylperoxymaléique et d'hydroxyde de calcium.

13. Une composition comme revendiqué dans l'une quelconque des revendications 1 à 12, caractérisée en ce qu'elle comprend de plus un ou plusieurs épaississants, agents de coloration, stabilisants limitant la vitesse de polymérisation et la poussée exothermique; stabilisants évitant le jaunissement, agents fluorescents et agents d'opacification radiologique.

14. Une restauration dentaire permanente préparée à partir d'une composition selon l'une quelconque des revendications 1 à 13.

15. Un procédé pour préparer une restauration dentaire permanente qui consiste à remplir un moule d'une composition selon l'une quelconque des revendications 1 à 13, laisser la composition faire prise ou provoquer sa prise, puis durcir la composition ou la laisser durcir.

16. Un procédé pour préparer des couronnes dentaires permanentes qui consiste à (I) mélanger (A) un premier composant comprenant 25 à 80 parties en poids d'un système monomère liquide comprenant du méthacrylate de méthyle, de 15 à 50 % en poids, par rapport au système monomère liquide, d'au moins un monomère réticulant à insaturation polyéthylénique; et un agent réducteur; avec (B) un second composant comprenant 20 à 75 parties en poids d'un mélange de (1) une substance particulaire organique polymère comprenant au moins un de: poly(éthylène-co-chlorotrifluoroéthylène), poly(fluorure de vinylidène), nylon et polyacétal, et (2) une substance particulaire minérale, le rapport pondéral de la substance particulaire organique polymère à la substance particulaire minérale étant de 1/5 à 1/1, les deux dites substances étant insolubles dans ledit premier composant, et un agent oxydant; (II) laisser le mélange obtenu atteindre une viscosité appropriée (III) remplir un moule préformé en forme de dent ou un porte-empreinte avec le mélange épaissi et (IV) appliquer le moule ou le porte-empreinte rempli au moignon de dent auquel la couronne doit être appliquée, (V) laisser la composition faire prise et (VI) retirer la couronne prise obtenue de la bouche puis du moule ou du porte-empreinte, la couronne, lorsqu'elle est durcie, ayant une résistance à la compression d'au moins 0,2068 GPa (30 000 psi) et/ou une usure par rotation d'au moins 20 h/25,4 $\mu$m (20 heures/mil).

17. Procédé comme revendiqué dans la revendication 16 appliqué à une composition selon l'une des revendications 3 à 13.